# EUROPEAN PATENT APPLICATION

(11) **EP 4 183 314 A1**
(43) Date of publication of application: **24.05.2023**
(21) Application number: 22207769.5
(22) Date of filing: 16.11.2022
(51) Int. Cl.: A61B 1/00, A61B 1/005, A61B 1/273, A61B 1/018

(54) **MEDICAL SYSTEM AND CONTROL METHOD OF MEDICAL SYSTEM**

(30) Priority: 18.11.2021 US 202163280716 P; 29.12.2021 US 202163294453 P
(71) Applicant: Olympus Corporation, Hachioji-shi Tokyo 192-8507 (JP)
(72) Inventor: INOUE, Shintaro, Center Valley, 18034 (US); GONO, Kazuhiro, Tokyo, 192-8507 (JP); MATSUO, Nobuko, Tokyo, 192-8507 (JP); OTAWARA, Takashi, Tokyo, 192-8507 (JP); MINAMIMOTO, Kazuki, Tokyo, 192-8507 (JP); PIROZZI, Anthony, Center Valley, 18034 (US)
(74) Representative: Schicker, Silvia

(57) **Abstract**

The medical system (10) includes an endoscope (100) and a control device (600). The endoscopic operation of the endoscope is electrically driven, and an endoscope image is captured. The endoscopic operation is at least one of forward and backward movement of an insertion section, a curving angle of a curving section of the insertion section, and rolling rotation of the insertion section. The control device controls the electrically-driven endoscopic operation. After the first positioning for positioning the insertion section with respect to the papillary portion of the duodenum is performed, the control device controls the electrically-driven endoscopic operation based on the endoscope image, thereby performing second positioning for positioning the distal end section of the insertion section with respect to the papillary portion.

## Description

### BACKGROUND

A technique called endoscopic retrograde cholangiopancreatography (ERCP) has been known that captures an X-ray image or a CT image of biliary duct by inserting a cannula into a biliary duct from a treatment tool channel of an endoscope, injecting a contrast agent from the cannula, and performing X-ray imaging or CT imaging. U.S. Patent Application Publication No. 2017/0086929 discloses an example in which a robotic catheter system for performing procedures by remotely operating a catheter system is applied to ERCP.

### SUMMARY

An embodiment of the present disclosure relates to
a medical system including:
an endoscope being configured to electrically drive an endoscopic operation, which is at least one of forward and backward movement of an insertion section, a curving angle of a curving section of the insertion section, and rolling rotation of the insertion section, and capture an endoscope image; and
a control device being configured to control the electrically-driven endoscopic operation,
   the control device controlling the electrically-driven endoscopic operation based on the endoscope image after first positioning for positioning the insertion section with respect to a papillary portion of duodenum so as to perform second positioning for positioning a distal end section of the endoscope with respect to the papillary portion.

Another embodiment of the present disclosure relates to
a control method of a medical system using an endoscope that electrically drives an endoscopic operation, which is at least one of forward and backward movement of an insertion section, a curving angle of a curving section of the insertion section, and rolling rotation of the insertion section, and captures an endoscope image,
the control method of the medical system including,
a step of the medical system inserting the insertion section of the endoscope into a body;
a step of the medical system performing first positioning for positioning the insertion section with respect to a papillary portion of duodenum;
after the step of the medical system performing the first positioning, a step of the medical system performing second positioning for positioning a distal end section of the endoscope with respect to the opening of the luminal tissue by controlling the electrically-driven endoscopic operation based on the endoscope image; and
after the step of the medical system performing the second positioning, a step of the medical system performing cannulation from the papillary portion to a biliary duct.

### BRIEF DESCRIPTION OF THE DRAWINGS

FIG. 1 shows organs and tissues involved in the ERCP procedure.
FIG. 2 shows a flow of the ERCP procedure.
FIG. 3 shows a schematic diagram of the form of papillary portion viewed directly from the front, and examples of individual differences in shape.
FIG. 4 is a cross-sectional view showing the form of a luminal tissue and its opening.
FIG. 5 shows a basic configuration example of a medical system according to the present embodiment.
FIG. 6 shows a first flow of the procedure according to the present embodiment.
FIG. 7 shows a comparison between a case without an overtube and a case with an overtube.
FIG. 8 shows the vicinity of the distal end of an endoscope positioned by an overtube and a balloon.
FIG. 9 shows a detailed configuration example of a medical system.
FIG. 10 shows a detailed configuration example of a drive control device.
FIG. 11 is a schematic view of an endoscope including a curving section and a driving mechanism thereof.
FIG. 12 shows a detailed configuration example of a forward/backward drive device.
FIG. 13 is a perspective view of a connecting section including a rolling drive device.
FIG. 14 shows a detailed configuration example of a distal end section of an endoscope including a raising base of a treatment tool.
FIG. 15 shows a detailed configuration example of a treatment tool.
FIG. 16 shows a configuration example of a drive system of an overtube.
FIG. 17 shows a configuration example of a drive system of a balloon.
FIG. 18 shows a first modification of a holding member.
FIG. 19 shows a second modification of a holding member.
FIG. 20 shows an organ structure of a patient who had a gastric bypass surgery according to the Roux-en Y method.
FIG. 21 shows a modification of an operation device for manually operating an electric endoscope.

### DESCRIPTION OF EXEMPLARY EMBODIMENTS

The following disclosure provides many different embodiments, or examples, for implementing different features of the provided subject matter. These are, of course, merely examples and are not intended to be limiting. In addition, the present disclosure may repeat reference numerals and/or letters in the various examples. This repetition is for the purpose of simplicity and clarity and does not in itself dictate a relationship between the various embodiments and/or configurations discussed. Further, when a first element is described as being "connected" or "coupled" to a second element, such description includes embodiments in which the first and second elements are directly connected or coupled to each other, and also includes embodiments in which the first and second elements are indirectly connected or coupled to each other with one or more other intervening elements in between.

### 1. Explanation of ERCP

The present embodiment relates to automatic control when performing ERCP using an electric medical system. ERCP stands for Endoscopic Retrograde Cholangiopancreatography. First, before describing the present embodiment, the details of procedure of ERCP is described below.

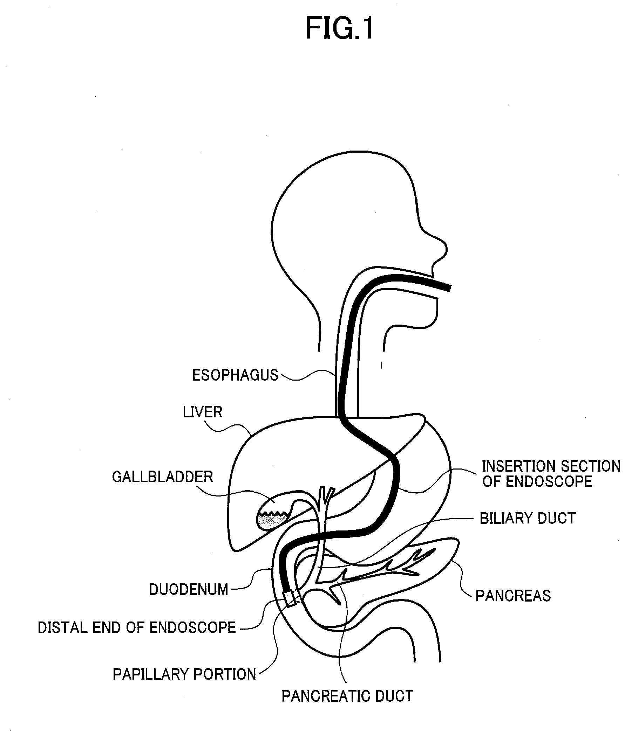

FIG. 1 shows organs and tissues involved in the ERCP procedure. The organs include a multiple types of tissues, forming a unique structure with a specific function. In FIG. 1, the liver, gallbladder, pancreas, esophagus, stomach, and duodenum are shown as organs. Tissues are formed by related cells combined, and examples include blood vessels, muscles, skin, and the like. In FIG. 1, a biliary duct and a pancreatic duct are shown as tissues.

The biliary duct is the target of the ERCP procedure. The biliary duct is a pipeline for allowing the bile produced in the liver to flow into the duodenum. When approaching the biliary duct using an endoscope, a treatment tool inserted into the channel of the endoscope is inserted to the biliary duct from the papillary portion of the duodenum while holding the endoscope at the position of the duodenum. Hereinafter, the papillary portion of the duodenum is simply referred to as a papillary portion. The papillary portion is a region including an opening of the luminal tissue with respect to the duodenum. Not only the opening but also the structure around the opening is referred to as a papillary portion. The opening of the luminal tissue is the opening of a common duct with respect to the duodenum. The common duct is formed as the confluence of the biliary duct and pancreatic duct. However, as described later, the papillary portion largely varies between individuals. For example, in some cases, the biliary duct opens directly to the duodenum without being merged with the pancreatic duct. In this case, the opening of the luminal tissue is the opening of the biliary duct.

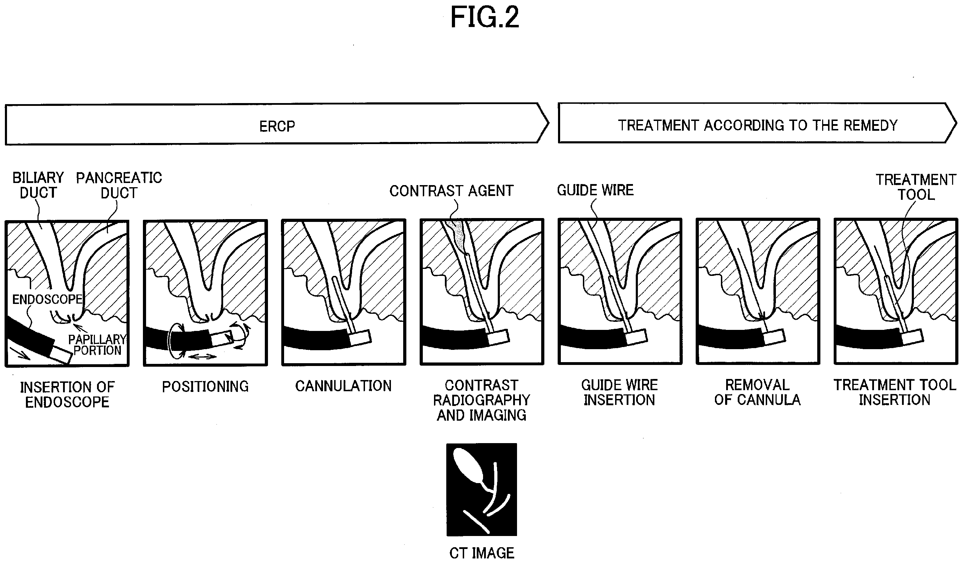

FIG. 2 shows a flow of the ERCP procedure. In ERCP, a side-viewing type endoscope in which a camera, an illumination lens, and an opening of a treatment tool channel are provided on a side surface of a distal end section of the endoscope is used. The camera is also referred to as an imaging device.

In the endoscope insertion step, the insertion section of the endoscope is inserted from the mouth to the duodenum through the esophagus and stomach. At this time, the insertion section is inserted until the papillary portion becomes roughly visible in the field of view of the endoscope. Next, in the positioning step, the position of the endoscope is adjusted relative to the papillary portion. Specifically, the position of the distal end section of the endoscope is adjusted so that the papillary portion is within the imaging range of the camera of the endoscope. Alternatively, the position of the distal end section of the endoscope is adjusted so that the camera of the endoscope is facing directly front of the papillary portion and the papillary portion appears in the center of the field of view.

Then, in the cannulation step, a cannula is inserted from the papillary portion into the biliary duct. Specifically, the cannula is inserted into the treatment tool channel of the endoscope so that the cannula protrudes from the channel opening of the distal end section of the endoscope. The distal end of the cannula is inserted into the common duct from the opening of the common duct, and the cannula is further inserted through the confluence of the biliary duct and the pancreatic duct toward the direction of the biliary duct. Cannulation refers to insertion of a cannula into a body. A cannula is a medical tube that is inserted into a body for medical purposes.

Next, in the contrast radiography and imaging step, a contrast agent is injected into the cannula and poured into the biliary duct through the distal end of the cannula. By performing X-ray or CT imaging in this state, an X-ray image or a CT (Computed Tomography) image showing the biliary duct, gallbladder, and pancreatic duct can be obtained. The procedure of ERCP has been described. After the procedure, various treatments are performed according to the results of diagnosis based on the X-ray image or CT image. An example of the treatment is described below.

In a guide wire insertion step, a guide wire is inserted into a cannula so that the guide wire is protruded from the distal end of the cannula, and the guide wire is inserted into the biliary duct. In a cannula removing step, the cannula is removed while leaving the guide wire inside the biliary duct. As a result, only the guide wire protrudes from the distal end section of the endoscope, indwelling in the biliary duct. Next, in a treatment tool insertion step, the treatment tool is inserted into the biliary duct along the guide wire. An example of a treatment tool is a basket or stent. The basket is used with a catheter. While allowing the guide wire to pass through the catheter, the catheter is inserted into the biliary duct along the guide wire. A basket made of a plurality of metal wires is inserted into the biliary duct from the distal end of the catheter, an object to be removed, such as a gallstone, is placed in the basket and held, and the object to be removed is taken out from the biliary duct by removing the basket and catheter in this state from the biliary duct. A stent is also used in a similar manner with a catheter and inserted into the biliary duct from the distal end of the catheter. The narrow portion of the biliary duct can be widened by inserting a stent; further, by keeping the stent therein, the narrow portion is held in a widened state by the indwelling stent.

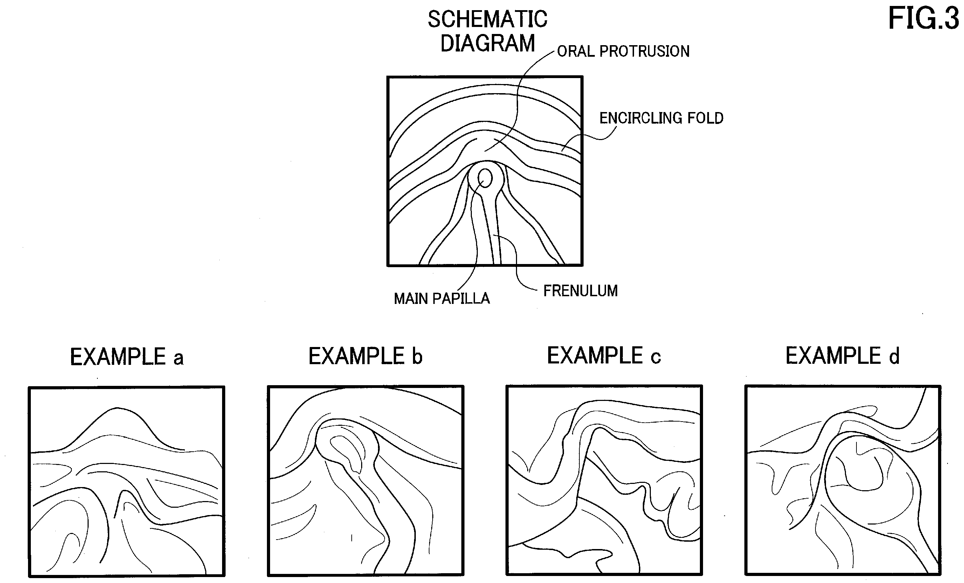

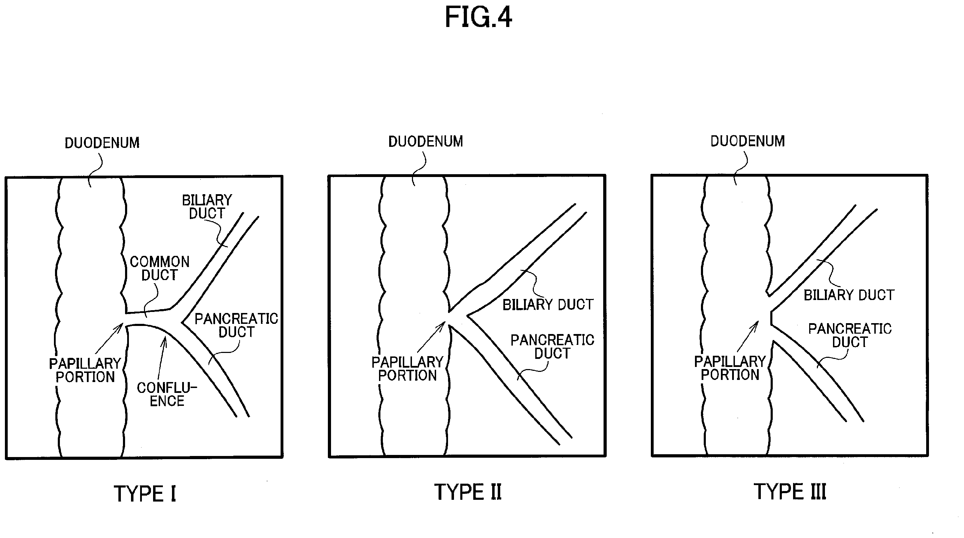

The ERCP procedure is performed as described above; however, in the positioning step, the procedure becomes difficult due to the individual differences in the papillary portions or luminal tissues. The following describes this issue with reference to FIGS. 3 and 4.

FIG. 3 is a diagram schematically showing the form of the papillary portion as viewed directly from the front thereof, and examples of individual differences of the papillary portion. As shown in the schematic diagram, structures peculiar to the papillary portion are present around the main papilla, which is the opening of the luminal tissue. Specifically, structures called frenulum, encircling fold, and oral protrusion are present around the main papilla.

Although the schematic diagram shows a typical form of the papillary portion, as shown in Examples a to d, the form of the papillary portion varies between individuals and different for each patient. Examples of the differences include unclear main papilla, frenulum, encircling fold, or oral protrusion, and other forms significantly different from the typical form. In addition, the opening of the luminal tissue is often closed; in this case, it is difficult to precisely recognize the opening by visual observation.

FIG. 4 is a cross-sectional view showing the form of the luminal tissue and its opening. The forms of the luminal tissue and its opening are classified into Type I(Y-shaped), Type II(V-shaped), and Type III(U-shaped or separated). In Type I, a biliary duct and a pancreatic duct merge into a common duct at the confluence thereof, and the common duct opens to the papillary portion. In Type II, the biliary duct and the pancreatic duct open to the papillary portion at the confluence thereof, and there is no common duct. In Type III, the biliary duct and the pancreatic duct are separately open to the papillary portion and there are no confluence or common duct. Type I is most common, but there are also Type II and Type III patients.

When cannulation into the biliary duct is performed, it is basically performed by referring to an endoscope image showing the papillary portion, as shown in Examples a to d in FIG. 3. As described with reference to FIGS. 3 and 4, there are various forms of papillary portion and luminal tissue, and it is difficult to specify the insertion position and insertion direction of the cannula from the endoscope image.

On the other hand, the operator estimates the position of the opening and the travelling direction of the biliary duct based on past cases, experiences, and the like while viewing the endoscope image, and tries to insert the cannula from the opening into the biliary duct according to the estimation. At this time, in order to more accurately estimate the position of the opening and the travelling direction of the biliary duct, it is desirable that the position of the papillary portion in the image and the angle of view of the image are easy to compare with those in the past cases or are familiar to the operator.

As shown in FIG. 1, such positioning of the endoscope is performed by operating the distal end of the endoscope insertion section reaching the duodenum from outside the body. However, since the insertion section and the organ through which the insertion section passes are flexible, the operation performed at the base end of the insertion section is not easily transmitted to the distal end section. In addition, since the distal end section of the endoscope is not fixed to the duodenum and floats in the air, the distal end section of the endoscope is not stable with respect to the papillary portion, and the positional relationship between the distal end section and the papillary portion is not easily determined. For these reasons, it is difficult to adjust the position of the distal end section of the endoscope so that the field of view of the endoscope is facing directly front of the papillary portion or so that the papillary portion appears in the center of the field of view.

### 2. Procedure Flow and Medical System According to the Present Embodiment

Therefore, in the present embodiment, the above-described positioning is automated by an electric medical system to assist the ERCP procedure. Further, by adding a configuration in which the insertion section of the endoscope is held in the duodenum, the electrically-driven force can be easily transmitted to the distal end section of the endoscope and the position of the distal end section can be desirably controlled. The details of this structure are described below.

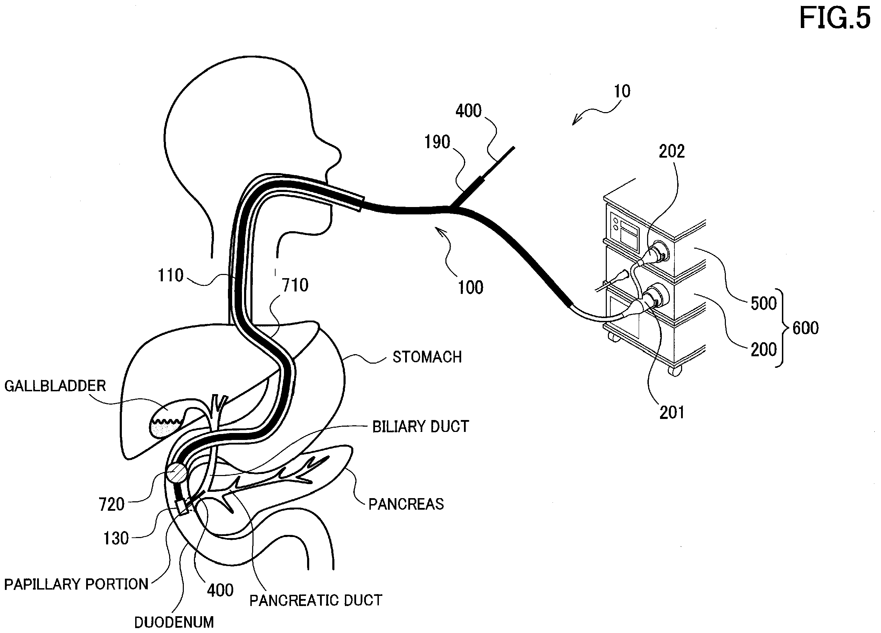

FIG. 5 shows a basic configuration example of a medical system 10 according to the present embodiment. The medical system 10 includes an endoscope 100, an overtube 710, a balloon 720, a treatment tool 400, and a control device 600. The medical system 10 is also referred to as an endoscope system or an electric endoscope system.

The overtube 710 is a tube with a variable hardness that covers the insertion section 110 of the endoscope 100. The balloon 720 is provided near the distal end on the outer side of the overtube 710. When the endoscope 100 and the overtube 710 are inserted into the body, at least the curving section of the insertion section 110 is exposed from the distal end of the overtube 710. The curving section refers to a section structured to be bent at an angle corresponding to the curving operation in the vicinity of the distal end of the insertion section 110. The base end of the overtube 710 is present outside the body. The base end side of the insertion section 110 is exposed from the base end of the overtube 710.

An insertion opening 190 of the treatment tool is provided at the base end side of the insertion section 110, and a treatment tool channel for allowing the treatment tool 400 to pass through from the insertion opening 190 to the opening of the distal end section 130 is provided inside the insertion section 110. The insertion opening 190 of the treatment tool is also called a forceps opening; however, the treatment tool to be used is not limited to forceps.

The endoscope 100 is detachably connected to a control device 600 using connectors 201 and 202. The control device 600 includes a drive control device 200 to which the connector 201 is connected, and a video control device 500 to which the connector 202 is connected. The drive control device 200 controls the electrical driving of the endoscope 100 via the connector 201. Although not shown in FIG. 5, an operation device for manually operating the electrical driving may be connected to the drive control device 200. The video control device 500 receives an image signal from a camera provided at the distal end section 130 of the endoscope 100 via the connector 202, generates a display image from the image signal, and displays it on a display device (not shown). In FIG. 5, the drive control device 200 and the video control device 500 are shown as separate devices, but they may be structured as a single device. In this case, the connectors 201 and 202 may be integrated into a single connector.

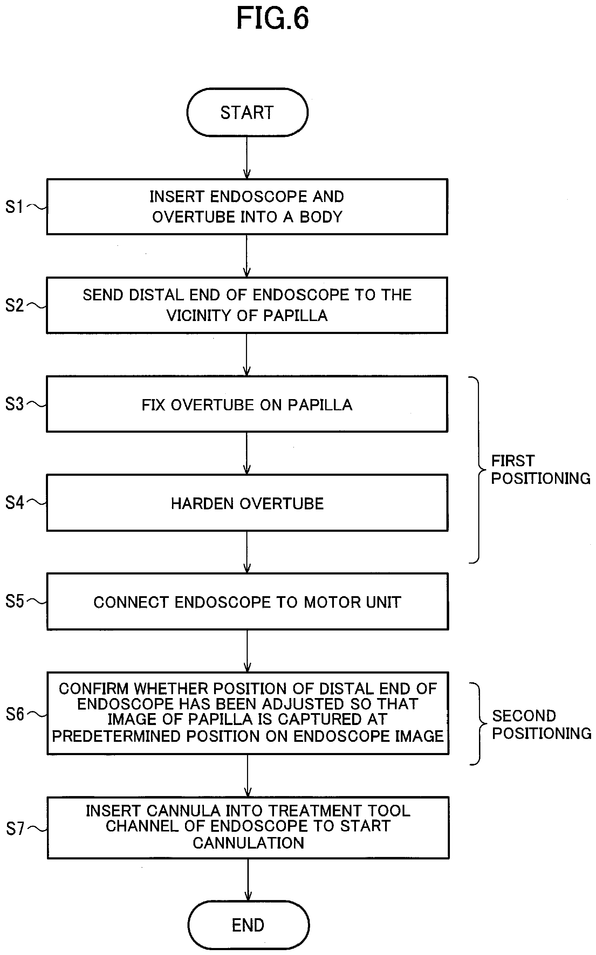

FIG. 6 shows a first flow of the procedure in the present embodiment. Here, an electric endoscope is assumed in which the forward and backward movement of the insertion section 110 of the endoscope 100, the curving of the curving section of the insertion section 110, and the rolling rotation of the insertion section 110 are electrically driven. However, it is sufficient that at least one of these functions is electrically driven. The term "electrical driving" means that the endoscope is driven by a motor or the like based on an electrical signal for controlling the endoscopic operation. For example, when the electrical driving is manually operated, an operation input to the operation device is converted into an electrical signal, and the endoscope is driven based on the electrical signal. In the following, the forward and backward movement may be simply referred to as "forward/backward movement".

In step S1, the operator inserts the insertion section 110 of the endoscope 100 and the overtube 710 into the duodenum. More specifically, in a state where the insertion section 110 is inserted into the overtube, the insertion section 110 and the overtube 710 are inserted into the duodenum together. The overtube 710, which is changeable in hardness, is soft in step S1. For example, the operator can move the insertion section 110 and the overtube 710 forward by a non-electrically-driven manual operation so that they are inserted into the body. The non-electrical driving means that the endoscope 100 is not electrically driven by a motor or the like, instead, the force applied to the operation section is directly transmitted to the endoscope by a wire or the like, thereby operating the endoscope. For example, in the present embodiment, steps S1 to S4 are not electrically driven. In this case, it is sufficient that at least the forward/backward movement is not electrically driven, and the curving, the rolling rotation, or both may be manually operated by electrical driving.

In step S2, the operator inserts the insertion section 110 until the distal end section 130 reaches the vicinity of the papillary portion. For example, when the operator manually inserts the insertion section 110 by non-electrical driving, the operator inserts the insertion section 110 until the papillary portion becomes visible in the endoscope image. At this point, the distal end of the endoscope 100 does not need to accurately reach the papillary portion; the distal end of the endoscope 100 may reach a position before the papillary portion or past the papillary portion.

In step S3, the operator fixes the distal end of the overtube 710 to the duodenum. As an example, the operator performs an operation to inflate the balloon 720 provided near the distal end of the overtube 710, and fixes the distal end of the overtube 710 to the duodenum by the balloon 720. In step S4, the operator performs an operation to harden the overtube 710. At this time, the overtube 710 is hardened while maintaining its shape in a state immediately before hardening, that is, the shape when it is inserted from the mouth to the duodenum. As a result, the insertion section 110 is held by the hardened overtube 710 and the balloon 720, thereby fixing the insertion route of the insertion section 110. These steps S3 and S4 are referred to as first positioning.

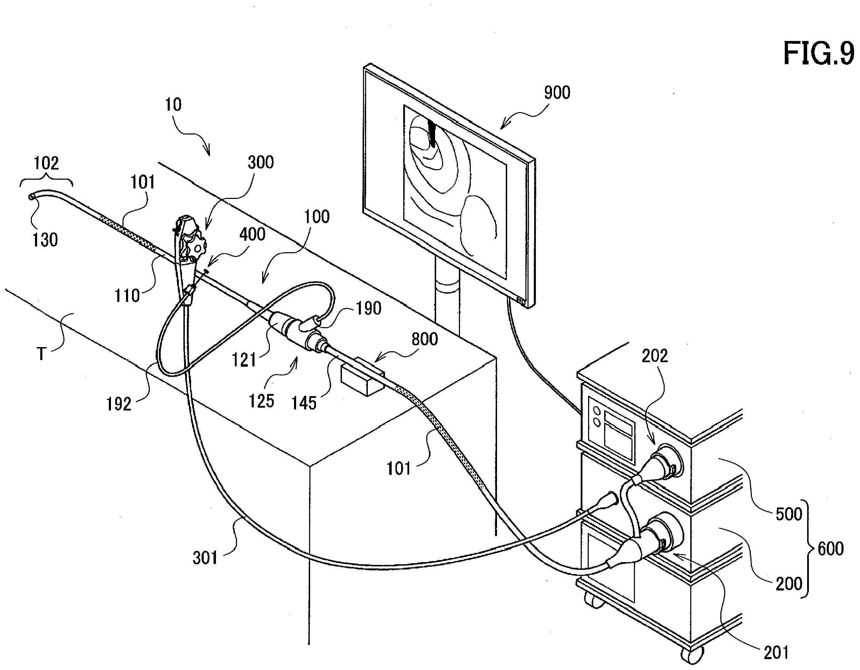

In step S5, the endoscope 100 is connected to the motor unit, and the non-electrical driving is switched to the electrical driving. The method of switching between the non-electrical driving and the electrical driving varies depending on the configuration of the drive mechanism. For example, when the medical system 10, which is described later with reference to FIG. 9, is used, in steps S1 to S4, the forward/backward movement is non-electrically driven and the curving and the rolling rotation are electrically driven. In this case, the forward/backward movement may be switched from the non-electrical driving to the electrical driving by connecting the endoscope 100 to the forward/backward drive device 800. Further, when the curving operation by non-electrical driving is enabled by providing a curving operation dial or the like capable of non-electrically performing the curving operation, the curving movement may be switched from the non-electrical driving to the electrical driving, for example, by connecting the connector 201 to the drive control device 200. Alternatively, even if the motor unit is kept connected, the motor may be structured to be detachable by a clutch mechanism or the like, and the non-electrical driving may be switched to the electrical driving by the clutch mechanism. Step S5 may be performed before step S1. For example, when the forward/backward movement is manually operated by electrical driving, the endoscope 100 may be connected to the motor unit before step S1.

In step S6, the drive control device 200 automatically positions the distal end section 130 at the papillary portion, and the operator confirms that the position of the distal end section 130 has been adjusted so that the papillary portion is captured at a predetermined position on the endoscope image. The drive control device 200 acquires an endoscope image from the video control device 500 and performs positioning of the distal end section 130 of the endoscope 100 based on the endoscope image. More specifically, the drive control device 200 controls the forward/backward movement, curving, or rolling rotation by electrical driving so that the papillary portion is captured at a position registered in advance on the endoscope image. The position registered in advance is, for example, the center of the image. More preferably, the positioning may be performed so that the opening of the luminal tissue is captured at a position registered in advance. Further, the drive control device 200 may perform electrical driving control based on the endoscope image so that the camera faces directly the front of the papillary portion or so that the papillary portion is captured at an appropriate angle of view. The drive control device 200 may also adjust the angle of view in imaging the papillary portion by controlling the diameter of the balloon 720 by electrical driving based on the endoscope image so that the distance between the camera and the papillary portion can be changed without changing the line-of-sight direction of the camera. This step S6 is referred to as second positioning.

In step S7, the operator inserts a cannula into the treatment tool channel through the insertion opening 190 to start cannulation into the biliary duct.

In FIG. 6, although the operation of the balloon in step S3 and the hardening of the overtube in step S4 are performed by non-electrical driving, they may be performed by electrical driving. In this case, the operator inputs an instruction from the operation device, and the drive control device 200 may inflate the balloon or harden the overtube by electrical driving using the instruction as a trigger. Alternatively, the drive control device 200 may perform an image recognition process for detecting the papillary portion from the endoscope image, and may automatically inflate the balloon or harden the overtube using the detection of the papillary portion from the endoscope image as a trigger.

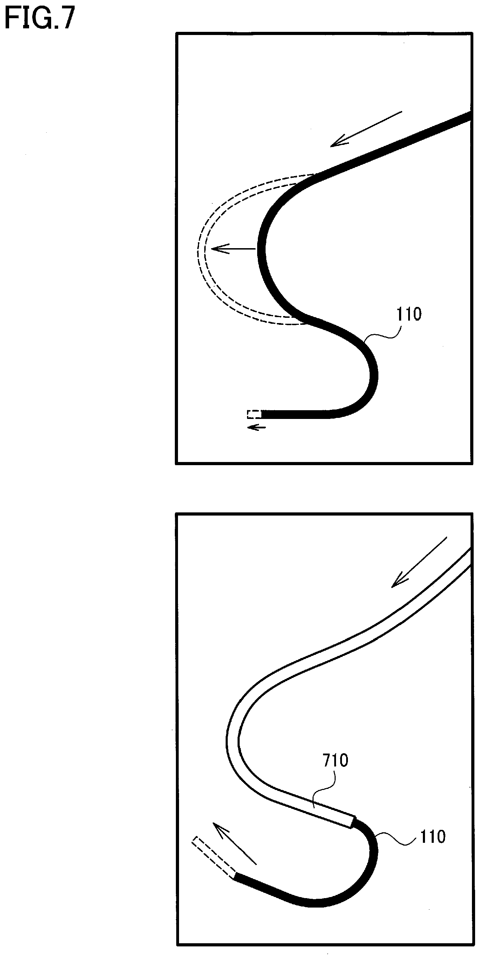

According to the procedure flow described above, by inflating the balloon 720 before hardening the overtube 710 in step S3, the position of the distal end of the overtube 710 does not shift when the overtube 710 is hardened. Specifically, the distal end of the overtube 710 can be accurately positioned. In addition, by the first positioning in steps S3 and S4, the insertion route of the insertion section 110 is held by the balloon 720 and the overtube 710. As a result, in the second positioning in step S6, the forward/backward movement, curving, or rolling rotation of the endoscope 100 due to the electrical driving is easily transmitted from the base end side to the distal end of the insertion section 110. This effect is described below with reference to FIG. 7.

FIG. 7 shows a comparison between a case without an overtube and a case with an overtube 710. The forward movement of the insertion section 110 is described herein as an example. The forward movement of the insertion section 110 is achieved by pushing the insertion section 110 in the axial direction by a slider mechanism or the like, which is described later. As shown in the upper figure, in the case where the insertion section 110 is not covered with the overtube 710, when the base end side of the insertion section 110 is pushed in the axial direction, the force is absorbed by the deformation of the insertion section 110 and is not easily transmitted to the distal end of the insertion section 110. This is because of the flexibility of the insertion section 110 and the stomach or the duodenum through which the insertion section 110 passes. As shown in the diagram below, in the case where the insertion section 110 is covered with the hardened overtube 710, when the base end side of the insertion section 110 is pushed in the axial direction, the insertion section 110 is moved forward in the overtube 710 using the hardened overtube 110 as a guide. As a result, the forward driving of the base end side is efficiently transmitted to the distal end section of the insertion section 110. Also for the curving or rolling rotation, the electrical driving from the base end side is efficiently transmitted to the distal end section of the insertion section 110 by holding the insertion section 110 by the overtube 710 and the balloon 720.

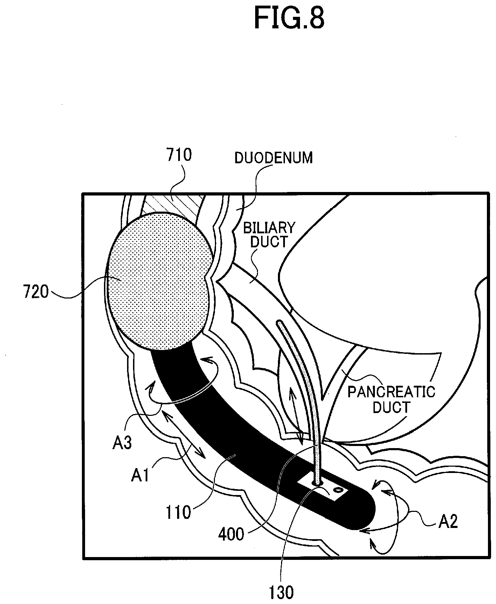

FIG. 8 shows the vicinity of the distal end of an endoscope positioned by the overtube 710 and the balloon 720. As shown in FIG. 8, the balloon 720 is fixed at a position slightly apart from the papillary portion to the pyloric side of the stomach. More specifically, the balloon 720 is positioned closer to the base end of the insertion section 110 than the base end of the curving section of the insertion section 110. By combining such a balloon 720 with the overtube 710 having a variable hardness, the curving section exposed to the papillary portion side from the balloon 720 and the distal end section 130 can be freely operated without being fixed, and the electrical driving from the base end side can be efficiently transmitted to the distal end section 130 of the endoscope.

The endoscopic operation by the electrical driving is the forward and backward movement shown in A1, a curving movement shown in A2, or a rolling rotation shown in A3. The forward movement is a shift toward the distal end side along the axial direction of the insertion section 110, and the backward movement is a shift toward the base end side along the axial direction of the insertion section 110. The curving movement is a movement by which the angle of the distal end section 130 is changed due to the bending of the curving section. The curving movement includes curving movements in two orthogonal directions, which can be controlled independently. One of the two orthogonal directions is referred to as the vertical direction and the other is referred to as the horizontal direction. The rolling rotation is a rotation about an axis of the insertion section 110.

FIG. 8 shows an example in which the balloon 720 is attached to the distal end of the overtube 710 and the endoscope protrudes from the distal end of the overtube 710. However, it is sufficient that the overtube 710 and the balloon 720 are configured so that a portion of the curving section beyond the base end can freely move. For example, it may also be arranged such that a soft tube with a constant hardness extends beyond the overtube with a variable hardness, and the balloon 720 is attached to the boundary thereof. In this case, although a part of the base end side of the curving section is covered with the soft tube, its movement is not hindered.

### 3. Detailed Configuration Example of Medical System

FIG. 9 shows a detailed configuration example of the medical system 10. The medical system 10 is a system for observing or treating the inside of the body of a patient lying on an operating table T. The medical system 10 includes an endoscope 100, a control device 600, an operation device 300, a treatment tool 400, a forward/backward drive device 800, and a display device 900. The control device 600 includes a drive control device 200 and a video control device 500.

The endoscope 100 is a device to be inserted into a lumen of a patient for the observation of an affected part. In this embodiment, the side to be inserted into a lumen of a patient is referred to as "distal end side" and the side to be attached to the control device 600 is referred to as "base end side". The endoscope 100 includes an insertion section 110, a connecting section 125, an extracorporeal soft section 145, and connectors 201 and 202. The insertion section 110, the connecting section 125, the extracorporeal soft section 145, and the connectors 201 and 202 are connected one another in this order from the distal end side.

The insertion section 110 is a portion to be inserted into a lumen of a patient, and is configured in a soft elongated shape. The insertion section 110 includes a curving section 102, an extracorporeal soft section for connecting the base end of the curving section 102 and the connecting section 125, and a distal end section 130 provided at the distal end of the curving section 102. An internal route 101 is provided inside the insertion section 110, the connecting section 125, and the extracorporeal soft section 145, and a curving wire passing through the internal route 101 is connected to the curving section 102. When the drive control device 200 drives the wire via the connector 201, the curving section 102 curves. Further, a raising base wire connected to the raising base provided at the distal end section 130 is connected to the connector 201 through the internal route 101. As the drive control device 200 drives the raising base wire, the raising angle of the treatment tool 400 protruding from the side surface of the distal end section 130 is changed. The side surface of the distal end section 130 is provided with a camera, an illumination lens, and an opening of a treatment tool channel. An image signal line for connecting the camera and the connector 202 is provided in the internal route 101, and an image signal is transmitted from the camera to the video control device 500 via the image signal line. The video control device 500 displays an endoscope image generated from the image signal on the display device 900.

The connecting section 125 is provided with an insertion opening 190 of the treatment tool and a rolling operation section 121. The treatment tool channel is provided in the internal route 101, one end of which is open to the distal end section 130 and the other end of which is open to the insertion opening 190 of the treatment tool. An extension tube 192 extending from the insertion opening 190 to the operation device 300 is connected to the insertion opening 190. The treatment tool 400 is inserted from an opening on the operation device 300 side of the extension tube 192, and protrudes to the opening of the distal end section 130 via the insertion opening 190 and the treatment tool channel. The extension tube 192 may be omitted, and the treatment tool 400 may be inserted through the insertion opening 190. The rolling operation section 121 is attached to the connecting section 125 so as to be rotatable about the axial direction of the insertion section 110. By rotating the rolling operation section 121, the insertion section 110 undergoes rolling rotation. As described later, the rolling operation section 121 can be electrically driven.

The forward/backward drive device 800 is a drive device for moving the insertion section 110 forward and backward by electrical driving. An extracorporeal soft section 140 is detachable from the forward/backward drive device 800, and an insertion section 110 moves forward and backward when the forward/backward drive device 800 causes the extracorporeal soft section 140 to slide in the axial direction in a state in which the extracorporeal soft section 140 is mounted on the forward/backward drive device 800. Although FIG. 9 shows an example in which the extracorporeal soft section 140 and the forward/backward drive device 800 are detachable, there is no such limitation, and it may be arranged such that the connecting section 125 and the forward/backward drive device 800 are detachable.

The operation device 300 is detachably connected to the drive control device 200 via an operation cable 301. The operation device 300 may communicate with the drive control device 200 through wireless communication instead of wired communication. When an operator operates the operation device 300, a signal of the operation input is transmitted to the drive control device 200 via the operation cable 301, and the drive control device 200 electrically drives the endoscope 100 to enable an endoscopic operation corresponding to the operation input based on the signal of the operation input. The operation device 300 has an operation input section having five or more channels corresponding to the forward and backward movement of the endoscope 100, the curving movements in two directions and the rolling rotation, and the operation of the raising base. If one or more of these operations are not electrically driven, the operation input section may be omitted. Each operation input section includes, for example, a dial, a joystick, a D-pad, a button, a switch, a touch panel, and the like.

The drive control device 200 electrically drives the endoscope 100 by driving a built-in motor based on an operation input to the operation device 300. Alternatively, when the motor is present outside the drive control device 200, the drive control device 200 transmits a control signal to the external motor based on an operation input to the operation device 300, thereby controlling the electrical driving. In addition, the drive control device 200 may drive a built-in pump or the like based on an operation input to the operation device 300, thereby causing the endoscope 100 to perform air supply suction. The air supply suction are performed through an air supply/suction tube provided in the internal route 101. One end of the air supply/suction tube opens to the distal end section 130 of the endoscope 100, while the other end is connected to the drive control device 200 via the connector 201. In addition, the treatment tool channel may be extended to the connector 201, and the treatment tool channel may also be used as an air supply/suction tube.

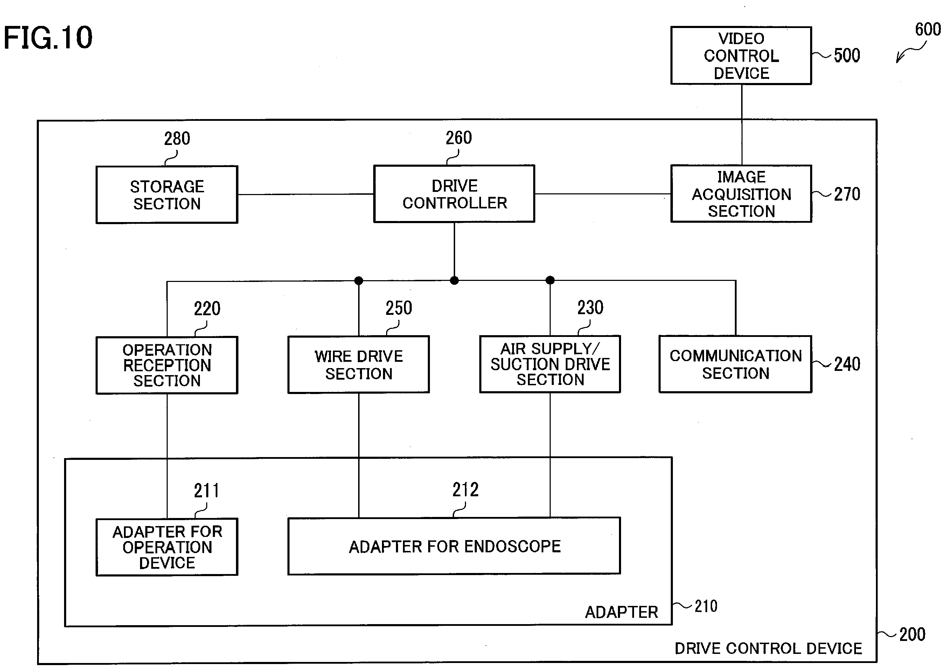

FIG. 10 shows a detailed configuration example of a drive control device 200. The drive control device 200 includes an image acquisition section 270, a storage section 280, a drive controller 260, an operation reception section 220, a wire drive section 250, an air supply/suction drive section 230, a communication section 240, and an adapter 210.

The adapter 210 includes an operation device adapter 211 to which the operation cable 301 is detachably connected, and an endoscope adapter 212 to which the connector 201 of the endoscope 100 is detachably connected.

The wire drive section 250 drives the curving movement of the curving section 102 of the endoscope 100 or the operation of the raising base of the treatment tool 400 based on the control signal from the drive controller 260. The wire drive section 250 includes a curving movement motor unit for driving the curving section 102 of the endoscope 100 and a raising base motor unit for driving the raising base. The endoscope adapter 212 has a curving movement coupling mechanism for enabling coupling to the curving wire on the endoscope 100 side. When the curving movement motor unit drives the coupling mechanism, the driving force is transmitted to the curving wire on the endoscope 100 side. Further, the endoscope adapter 212 has a raising base coupling mechanism for enabling coupling to the raising base wire on the endoscope 100 side. When the raising base motor unit drives the coupling mechanism, the driving force is transmitted to the raising base wire on the endoscope 100 side.

The air supply/suction drive section 230 drives air supply suction of the endoscope 100 based on a control signal from the drive controller 260. The air supply/suction drive section 230 is connected to an air supply/suction tube of the endoscope 100 via the endoscope adapter 212. The air supply/suction drive section 230 includes a pump or the like, and supplies air to the air supply/suction tube or sucks air from the air supply/suction tube 172.

The communication section 240 communicates with a drive device provided outside the drive control device 200. The communication may be wireless communication or wired communication. The drive device provided outside is a forward/backward drive device 800 for performing forward and backward movement, a rolling drive device for performing the rolling rotation, an overtube drive device for changing the hardness of the overtube 710, a balloon drive device for changing the diameter of the balloon 720 or the like.

The drive controller 260 controls the forward and backward movement, the curving movement and the rolling rotation of the endoscope 100, the raising angle of the treatment tool 400 made by the raising base, and the air supply suction by the endoscope 100. When the hardness control of the overtube 710 or the diameter control of the balloon 720 is electrically driven, the drive controller 260 performs the control thereof. The drive controller 260 is, for example, a processor such as a CPU (Central Processing Unit), a GPU (Graphics Processing Unit), a DSP (Digital Signal Processor), or the like. For example, the storage section 280 stores a computer-readable program, and the functions of the drive controller 260 are implemented as processes as the processor executes the program. However, the hardware of the drive controller 260 is not limited to that described above, and may be structured using circuits with various configurations.

The electric control performed by the drive controller 260 includes a manual mode in which the operator manually operates the electrical driving of the endoscope 100 or the like and an automatic control mode in which the electrical driving of the endoscope 100 or the like is automatically controlled based on an endoscope image. In the automatic mode of the present embodiment, the positioning step described with reference to FIG. 2 is automated. In the automatic mode, at least one of the forward and backward movement, the curving movement, and the rolling rotation of the endoscope 100 may be automated. That is, the raising angle of the treatment tool 400 by the raising base, the hardness control of the overtube 710, the diameter control of the balloon 720, the air supply suction of the endoscope 100, or a part of the forward and backward movement, the curving movement or rolling rotation of the endoscope 100 may be manually operated.

First, the manual mode is described below. The operation reception section 220 receives an operation input signal from the operation device 300 via the operation cable 301 attached to the operation device adapter 221. When the operation device 300 communicates with the drive control device 200 by wireless communication, the operation reception section 220 may be a wireless communication circuit.

The drive controller 260 controls the electrical driving based on an operation input signal from the operation reception section 220. Specifically, when the curving operation is performed, the drive controller 260 outputs a control signal indicating the curving direction or the curving angle to the wire drive section 250, and the wire drive section 250 drives the curving wire so that the curving section 102 curves in the curving direction or the curving angle. Also, when the forward and backward movement operation is performed, the drive controller 260 transmits a control signal indicating the forward/backward direction or the forward/backward movement amount to the forward/backward drive device via the communication section 240, and the forward/backward drive device moves the extracorporeal soft section 140 forward or backward so that the endoscope 100 moves forward or backward in the forward/backward direction or the forward/backward movement amount. Further, when the rolling rotation operation is performed, the drive controller 260 transmits a control signal indicating the rolling rotation direction or the rolling rotation angle to the rolling drive device via the communication section 240, and the rolling drive device performs rolling rotation of the insertion section 110 so that the endoscope 100 undergoes rolling rotation in the rolling rotation direction or at the rolling rotation angle. Similar controls are performed for other electrical driving.

Next, the automatic control mode is described below. The image acquisition section 270 is a communication interface for receiving image data of an endoscope image from the video control device 500 by wired communication or wireless communication. The image acquisition section 270 outputs image data of the received endoscope image to the drive controller 260.

The storage section 280 stores a reference image of the papillary portion that serves as a reference for positioning with respect to the papillary portion. The reference image is an image in which the papillary portion is captured such that the opening of the luminal tissue appears at a predetermined position. The predetermined position is, for example, the center of the image, and corresponds to the "position registered in advance" described above. The storage section 280 may store a plurality of reference images corresponding to the various forms described with reference to FIG. 3 or FIG. 4. The storage section 280 is a storage device such as a semiconductor memory or a magnetic storage device. The semiconductor memory may be a volatile memory such as a SRAM or a DRAM, or a nonvolatile memory such as an EEPROM.

The drive controller 260 controls the endoscopic operation or the diameter of the balloon 720 so that the position of the papillary portion in the endoscope image is brought close to the position of the papillary portion in the reference image. For example, the drive controller 260 extracts an image feature amount of the papillary portion from the endoscope image and the reference image, and determines the position of the papillary portion in the endoscope image based on the comparison result of the image feature amount. More preferably, the drive controller 260 controls the endoscopic operation or the diameter of the balloon 720 so that the position of the opening of the luminal tissue on the endoscope image comes close to a predetermined position. For example, a reference image in which the opening of the luminal tissue is located at a predetermined position is stored in the storage section 280. The drive controller 260 performs positioning of the opening of the luminal tissue by performing positioning of the papillary portion. Even in a case where the opening of the luminal tissue is closed and the opening cannot be recognized from the image, it is possible to perform the positioning so that the opening is present at a predetermined position in the image by performing positioning of the papillary portion. The storage section 280 may store the image feature amount extracted from the reference image, instead of the reference image.

The drive controller 260 may also control the endoscopic operation or the raising angle of the treatment tool 400 so that the distal end of the treatment tool 400 is directed toward the opening of the luminal tissue based on the endoscope image. Alternatively, the drive controller 260 may also control the endoscopic operation or the raising angle of the treatment tool 400 so that the distal end of the treatment tool 400 is directed toward the travelling direction of the biliary duct based on the endoscope image. For example, it may be arranged such that information on the travelling direction of the biliary duct is given to the reference image, and the distal end of the treatment tool 400 is controlled to be directed to the travelling direction of the biliary duct based on the information. Here, the travelling direction is a two dimensional direction on the endoscope image. That is, the endoscopic operation or the raising angle of the treatment tool 400 is controlled so that the travelling direction of the biliary duct and the direction to which the treatment tool 400 faces are substantially parallel to each other on the endoscope image. However, in a case where three dimensional information of the travelling direction of the biliary duct can be obtained from a CT image or the like, the control may be performed so that the travelling direction of the biliary duct and the direction to which the treatment tool 400 faces are substantially three dimensionally parallel.

Alternatively, the drive controller 260 may control the position of the opening of the luminal tissue in the endoscope image to be equal to the position registered in advance based on the result of the image recognition process using machine learning. More specifically, the storage section 280 stores a trained model, and the drive controller 260 performs the positioning control described above by performing a process based on the trained model. For example, the trained model receives input of an endoscope image and is trained to output information such as an endoscopic operation so that the position of the opening of the luminal tissue is located at a predetermined position in the endoscope image. Further, the trained model may be trained such that it receives input of an endoscope image and outputs information of the travelling direction of the biliary duct from the endoscope image. The drive controller 260 presumes information on an endoscopic operation or the like from an endoscope image by a process based on the trained model, and outputs a control signal for controlling the endoscopic operation or the raising angle of the treatment tool or the like to the wire drive section 250 or the like based on the information. In this example, the storage section 280 does not need to store the reference image or the image feature amount, and the "position registered in advance" of the opening of the luminal tissue is already reflected in the trained model by learning.

As described above, when cannulation into the biliary duct is performed, it is difficult to estimate the insertion position and the insertion direction of the cannula from the endoscope image of the papillary portion. In this regard, according to the present embodiment described above, in the second positioning after the first positioning by the overtube 710 or the like, the electrical driving of the endoscopic operation or the like is automatically controlled based on the endoscope image, so that the distal end section 130 of the endoscope 100 is automatically positioned with respect to papillary portion. As a result, it is not necessary to perform minute position adjustment by non-electric manual operation, and it is possible to assist the ERCP procedure performed by an inexperienced operator or the like. In addition, since the position of the papillary portion in the endoscope image is automatically controlled so as to be located at the position registered in advance by the automatic control, the operator can easily specify the insertion position and the insertion direction of the cannula from the endoscope image. For example, since the opening of the luminal tissue is automatically controlled so as to be at a predetermined position in the image, the operator can more easily grasp the position of the opening even when the opening cannot be visually recognized from the image.

### 4. Detailed Configuration Example of Each Part of Medical System

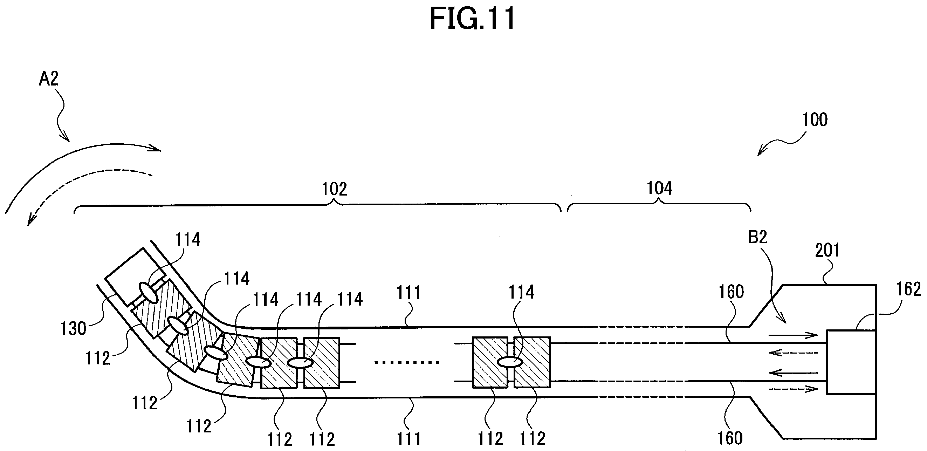

FIG. 11 is a schematic view of an endoscope 100 including a curving section 102 and a driving mechanism thereof. An endoscope 100 includes a curving section 102, a soft section 104, and a connector 201. The soft section 104 corresponds to the intracorporeal soft section and the extracorporeal soft section 145 described above with reference to FIG. 9. In FIG. 11, the connecting section 125 is omitted.

The curving section 102 and the soft section 104 are covered with an outer sheath 111. The inside of the tube of the outer sheath 111 corresponds to the internal route 101 in FIG. 9. The curving section 102 includes a plurality of curving pieces 112 and a distal end section 130 connected to the distal end of the curving pieces 112. Each of the plurality of curving pieces 112 and the distal end section 130 is connected in series from the base end side to the distal end side by a rotatable connecting section 114, thereby forming a multi-joint structure. The connector 201 is provided with a coupling mechanism 162 on the endoscope side connected to a coupling mechanism on the drive control device 200 side. By attaching the connector 201 to the drive control device 200, it is possible to electrically drive the curving movement. A curving wire 160 is provided in the outer sheath 111. One end of the curving wire 160 is connected to the distal end section 130. The curving wire 160 passes through the soft section 104 by penetrating through a plurality of curving pieces 112, turns back in a coupling mechanism 162, passes through the soft section 104 again, penetrates through the plurality of curving pieces 112. The other end of the curving wire 160 is connected to the distal end section 130. The driving force from the wire drive section 250 is transmitted to the curving wire 160 via the coupling mechanism 162 as the pulling force of the curving wire 160.

As shown by the solid line arrow B2, when the upper wire in the figure is pulled, the lower wire is pushed, whereby the multiple joints of the curving pieces 112 are bent upward in the figure. As a result, as indicated by the solid line arrow A2, the curving section 102 is curved upward in the figure. When the lower wire in the figure is pulled as indicated by the dotted arrow B2, similarly, the curving section 102 is curved downward in the figure as indicated by the dotted arrow A2. As described with reference to FIG. 8, the curving section 102 can be curved independently in two orthogonal directions. Although FIG. 11 shows a curving mechanism for one direction, two sets of curving wires are actually provided, and each curving wire can be curved independently in two directions by being pulled independently by the coupling mechanism 162.

Note that the mechanism for the electrically-driven curving is not limited to that described above. For example, a motor unit may be provided instead of the coupling mechanism 162. Specifically, it may be arranged such that the drive control device 200 transmits a control signal to the motor unit via the connector 201, and the motor unit drives the curving movement by pulling or relaxing the curving wire 160 based on the control signal.

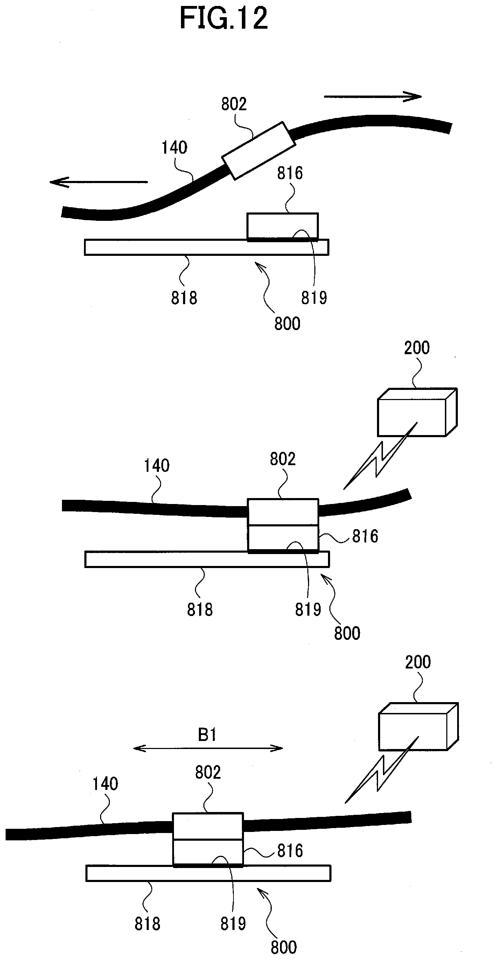

FIG. 12 shows a detailed configuration example of a forward/backward drive device 800. The forward/backward drive device 800 includes a motor unit 816, a base 818, and a slider 819.

As shown in the upper and middle figures, the extracorporeal soft section 140 of the endoscope 100 is provided with an attachment 802 detachable from the motor unit 816. As shown in the middle figure, the attachment of the attachment 802 to the motor unit 816 enables electrical driving of forward/backward movement. As shown in the lower figure, the slider 819 supports the motor unit 816 while enabling the motor unit 816 to move linearly with respect to the base 818. The slider 819 is fixed to the operating table T shown in FIG. 9. As shown in B1, the drive control device 200 transmits a forward or backward control signal to the motor unit 816 by wireless communication, and the motor unit 816 and the attachment 802 move linearly on the slider 819 based on the control signal. As a result, the forward and backward movement of the endoscope 100 shown in A1 in FIG. 8 is achieved. Note that the drive control device 200 and the motor unit 816 may be connected by wired connection.

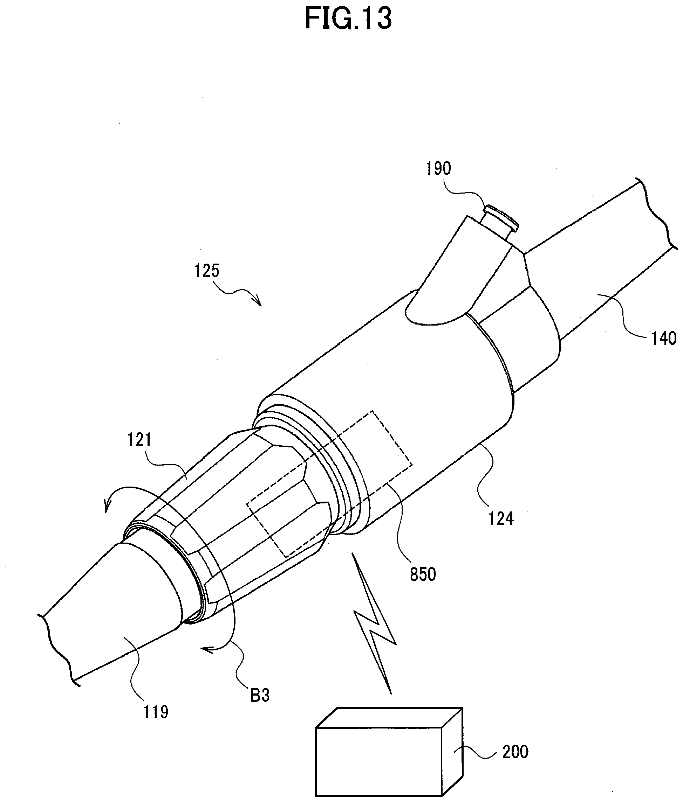

FIG. 13 is a perspective view of the connecting section 125 including a rolling drive device 850. The connecting section 125 includes a connecting section main body 124 and a rolling drive device 850.

The insertion opening 190 of the treatment tool is provided in the connecting section main body 124 and is connected to the treatment tool channel inside the connecting section main body 124. The connecting section main body 124 has a cylindrical shape, and a cylindrical member coaxial with the cylinder is rotatably provided inside the connecting section main body 124. The base end section of the intracorporeal soft section 119 is fixed to the outside of the cylindrical member, and the base end section serves as a rolling operation section 121. As a result, the intracorporeal soft section 119 and the cylindrical member can rotate with respect to the connecting section main body 124 about the axial direction of the intracorporeal soft section 119. The rolling drive device 850 is a motor unit provided inside the connecting section main body 124. As shown in B3, the drive control device 200 transmits a rolling rotation control signal to the rolling drive device 850 by wireless communication, and the rolling drive device 850 rotates the base end section of the intracorporeal soft section 119 with respect to the connecting section main body 124 based on the control signal, thereby causing rolling rotation of the intracorporeal soft section 119. As a result, the rolling rotation of the endoscope 100 shown in A3 in FIG. 8 is achieved. The rolling drive device 850 may include a clutch mechanism, and the rolling rotation may be switched between non-electrical driving and electrical driving by the clutch mechanism. The drive control device 200 and the rolling drive device 850 may be connected by wired connection via a signal line passing through the internal route 101.

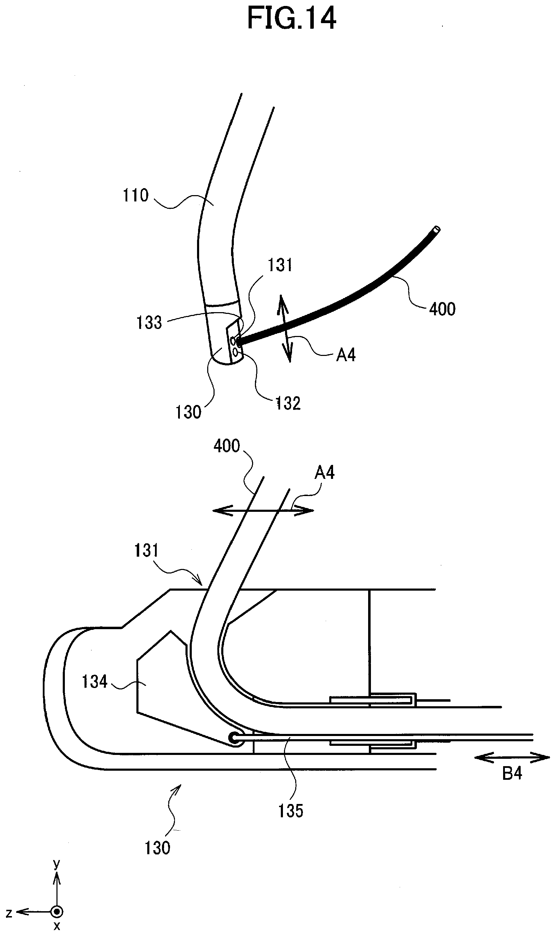

FIG. 14 shows a detailed configuration example of a distal end section 130 of an endoscope including a raising base of a treatment tool. The upper figure shows an external view of the distal end section 130. An opening 131 of a treatment tool channel, a camera 132, and an illumination lens 133 are provided on the side surface of the distal end section 130. As shown in the lower figure, the direction parallel to the axial direction of the distal end section 130 is defined as z direction, the direction parallel to the line-of-sight direction of the camera 132 is defined as y direction, and the direction orthogonal to the z direction and the y direction is defined as x direction. The lower figure shows a cross-sectional view of the distal end section 130 in a plane that is parallel to the yz plane of the treatment tool channel and that passes through the opening 131 of the treatment tool channel.

The distal end section 130 includes a raising base 134 and a raising base wire 135. The raising base 134 is swingable about an axis parallel to the x direction. One end of the raising base wire 135 is connected to the raising base 134, while the other end is connected to the drive control device 200 via the connector 201. As shown in B4, the wire drive section 250 of the drive control device 200 pushes and pulls the raising base wire 135 to swing the raising base 134, thereby, as shown in A4, changing the raising angle of the treatment tool 400. The raising angle is an angle of the treatment tool 400 protruding from the opening 131. The raising angle can be defined, for example, by an angle formed by the treatment tool 400 protruding from the opening 131 and the z direction.

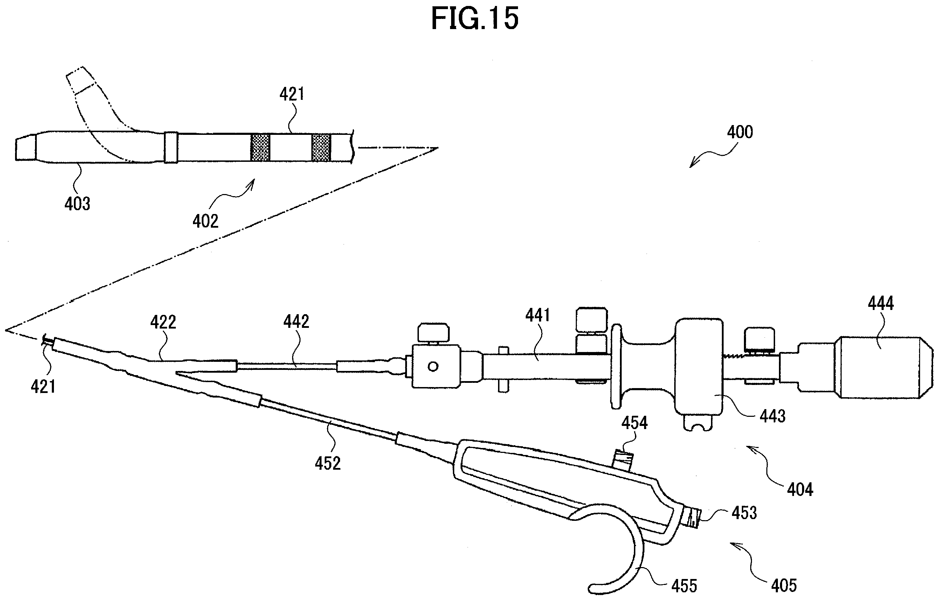

FIG. 15 shows a detailed configuration example of the treatment tool 400. Herein, as an example of the treatment tool 400, a cannula capable of operating curving of the distal end is shown. The treatment tool 400 includes a long-length insertion section 402 extending in the axial direction, a curving movement section 403 capable of curving movement, a first operation section 404 for operating the curving movement section 403, and a second operation section 405 for inserting a contrast agent or a guide wire.

The insertion section 402 has a tube 421, and the curving movement section 403 is connected to the distal end of the tube 421. In FIG. 15, the distal end side of the tube 421 is enlarged. The tube 421 is also referred to as a sheath. The operator holds the tube 421 of the treatment tool 400 inserted into the treatment tool channel of the endoscope 100, and pushes and pulls the tube 421 to move the treatment tool 400 forward and backward.

A connector 422 is connected to the base end of the tube 421. The first operation section 404 and the second operation section 405 are connected to the connector 422. The first operation section 404 includes a connecting tube 442, one end of which is connected to the connector 422, a first operation main body 441 connected to the other end of the connecting tube 442, a grip 444 fixed to the base end of the first operation main body 441, and a slider 443 provided movable forward and backward in the axial direction of the first operation main body 441. Inside the tube 421, the connector 422, the connecting tube 442, and the first operation main body 441, a wire for connecting the curving movement section 403 and the slider 443 is provided. When the operator pulls the slider 443 while holding the grip 444, the wire is pulled and the curving movement section 403 is curved.

The second operation section 405 includes a connecting tube 452, one end of which is connected to the connector 422, a second operation main body 451 connected to the other end of the connecting tube 452, a first opening 453 opened in the axial direction of the connecting tube 452 on the base end side of the second operation main body, a second opening 454 opened to the outer surface of the second operation main body 451, and a hook 455 provided on the second operation main body 451. The hook 455 has elasticity and is formed in a substantially C-shape, and is used for locking the treatment tool 400 to the endoscope 100 or the like. The first opening 453 and the second opening 454 are connected to the tube 421 via the second operation main body 451, the connecting tube 452, and the connector 422. By inserting a contrast agent or a guide wire from the first opening 453 or the second opening 454, the contrast agent can be injected into the body or the guide wire can be inserted into the body from the distal end of the treatment tool 400.

Although an example in which the treatment tool 400 is manually operated by non-electrical driving has been described herein, the operation of the treatment tool 400 may be operated by electrical driving. For example, using a method similar to the electrical driving of the endoscope 100, it is possible to perform the forward/backward movement of the treatment tool 400, the curving of the distal end, or the rolling rotation by electrical driving.

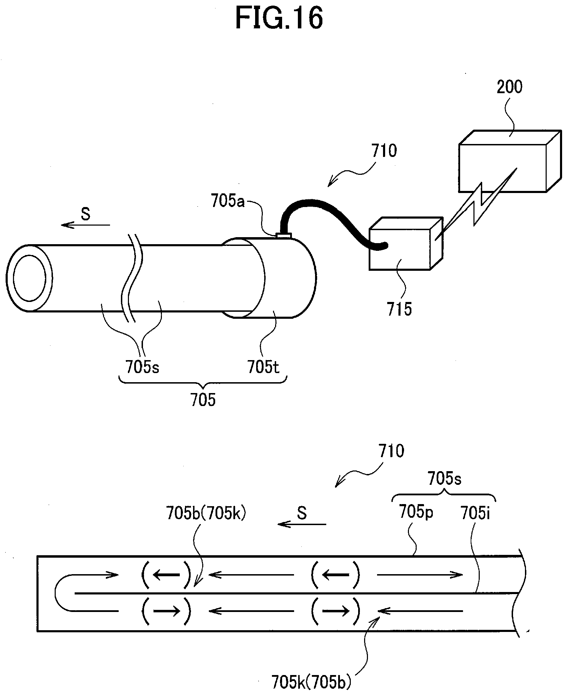

FIG. 16 shows a configuration example of the drive system 701 of the overtube 710. As shown in the upper figure, the drive system 701 includes an overtube 710 and an overtube drive device 715.

The overtube 710 is structured such that its hardness is variable, its shape is variable during softening, and its shape is maintained during hardening. Although an example of using a shape memory polymer with its hardness changeable according to the temperature is shown herein, the method of varying the hardness is not limited to this example, and, for example, a structure in which the hardness is varied using a multi-joint structure made of a plurality of bridge members connected in series may be used. As shown in the upper figure, the overtube 710 includes an insertion section 705s and an operation section 705t. The operation section 705t is provided with a connecting section 705a, and the overtube drive device 715 is connected via the connecting section 705a.

The lower figure shows a cross-sectional view of the insertion section 705s in a cross section parallel to the insertion direction S. Although there are two tube walls in the cross section, the figure shows only one of them. The other wall has a similar structure. The insertion section 705s includes a tube member 705p and a shape memory polymer tube 705i.

The shape memory polymer tube 705i is hardened when a fluid having a temperature lower than the glass transition temperature is supplied, and is softened when a fluid having a temperature higher than the glass transition temperature is supplied. The shape memory polymer tube 705i is covered with the tube member 705p, and a supply path 705k and a collection path 705b connected to the distal end of the supply path 705k are provided between the shape memory polymer tube 705i and the inner wall of the tube member 705p. The overtube drive device 715 is a fluid supply device including a pump and the like. The fluid supply device supplies a fluid of a predetermined temperature to the supply path 705k and collects the fluid from the collection path 705b. The drive control device 200 transmits a control signal for controlling the hardness of the overtube 710 to the overtube drive device 715 by wireless communication, and the overtube drive device 715 changes the hardness of the overtube 710 by setting the temperature of the fluid based on the control signal. In FIG. 16, in the overtube 710, the supply path 705k is located in the inner side and the collection path 705b is located in the outer side; however, the supply path 705k may be located in the outer side and the collection path 705b may be located in the inner side. Further, the drive control device 200 and the overtube drive device 715 may be connected by wired connection.

The mechanism for changing the hardness of the overtube by electrical driving is not limited to that described above. For example, it may be arranged such that a plurality of bridge members are connected in series, and the degree of contact between the bridge members are changed by electrical driving. Specifically, it may be arranged such that the overtube is softened by arranging adjacent bridge members so that they can be agitated, and the overtube is hardened by arranging adjacent bridge members so that they are in contact with each other and cannot be easily agitated.

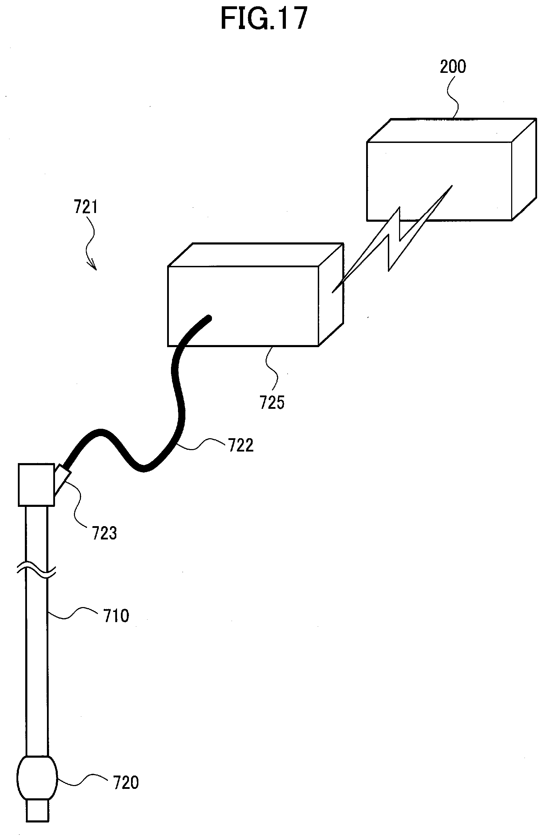

FIG. 17 shows a configuration example of the drive system 721 of the balloon 720. The drive system 721 includes a balloon 720, a connecting tube 722, and a balloon drive device 725.

The balloon 720 is made of an expandable member and is provided near the distal end of the overtube 710. The balloon 720 has a doughnut shape and is disposed so as to surround the outer periphery of the distal end section of the overtube 710. A balloon vent hole 723 is provided at the base end of the overtube 710, and the balloon vent hole 723 and the balloon 720 are connected to each other via a pipeline (not shown). The balloon vent hole 723 and the balloon drive device 725 are connected to each other by a connecting tube 722. The balloon drive device 725 includes a pump or the like, and inflates the balloon 720 by sending air to the balloon 720 through the connecting tube 722, or deflates the balloon 720 by sucking air from the balloon 720. The drive control device 200 transmits a control signal for controlling the diameter of the balloon 720 to the balloon drive device 725 by wireless communication, and the balloon drive device 725 inflates or deflates the balloon 720 based on the control signal, thereby controlling the diameter of the balloon 720. The drive control device 200 and the balloon drive device 725 may be connected by wired connection.

### 5. Modifications

Some modifications are described below. Each modification can be combined with any of the embodiments described above.

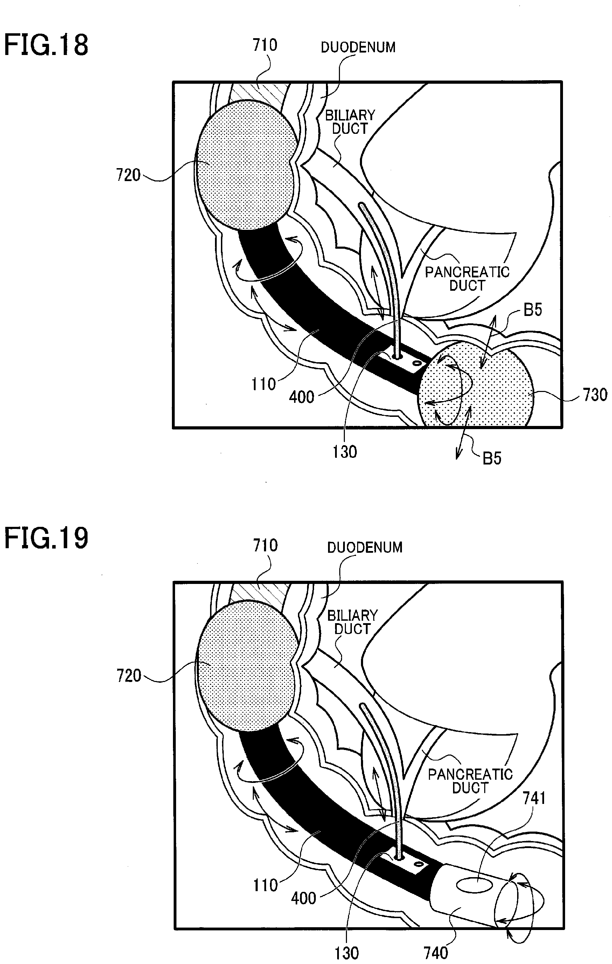

FIG. 18 shows a first modification of the holding member. In this modification, a balloon 730 is provided at the distal end section 130 of the endoscope 100. The diameter of the balloon 730 is variable by inflation and deflation. The diameter of the balloon 730 may be non-electrically controlled, or the diameter of the balloon 730 may be electrically controlled in a manner similar to that for the balloon 720. The electrical driving may be manual operation or automatic control based on an endoscope image. By fixing the distal end section 130 of the endoscope 100 to the duodenum by the balloon 730, the positional relationship between the distal end section 130 and the papillary portion can be stabilized during cannulation. As shown in B5, by adjusting the diameter of the balloon 730, it is possible to adjust the distance between the distal end section 130 and the papillary portion without changing the angle of the distal end section 130.

FIG. 19 shows a second modification of the holding member. In this modification, a suction cap 740 is provided at the distal end section 130 of the endoscope 100. A suction opening 741 is provided on the side surface of the suction cap 740 in the same direction as the line-of-sight direction of the camera or the like. By sucking air through the suction opening 741, the intestinal wall of the duodenum is drawn toward the distal end section 130. This allows adjustment of the distance between the distal end section 130 and the papillary portion. This suction may be performed by non-electrical driving or electrical driving. The electrical driving may be manual operation or automatic control based on an endoscope image.

As a third modification of the holding member, a basket may be used instead of the balloon 720 or 730. By expanding or contracting the basket made of a plurality of bundled wires, a function similar to that of the balloon is achieved. As a fourth modification of the holding member, a grasper or a retractor may be provided at the distal end section 130 of the endoscope 100. The grasper or the retractor grasps the intestinal wall of the duodenum and pushes or pulls the intestinal wall. As a result, the distal end section 130 is held in the duodenum, and the distance between the distal end section 130 and the papillary portion is adjusted.

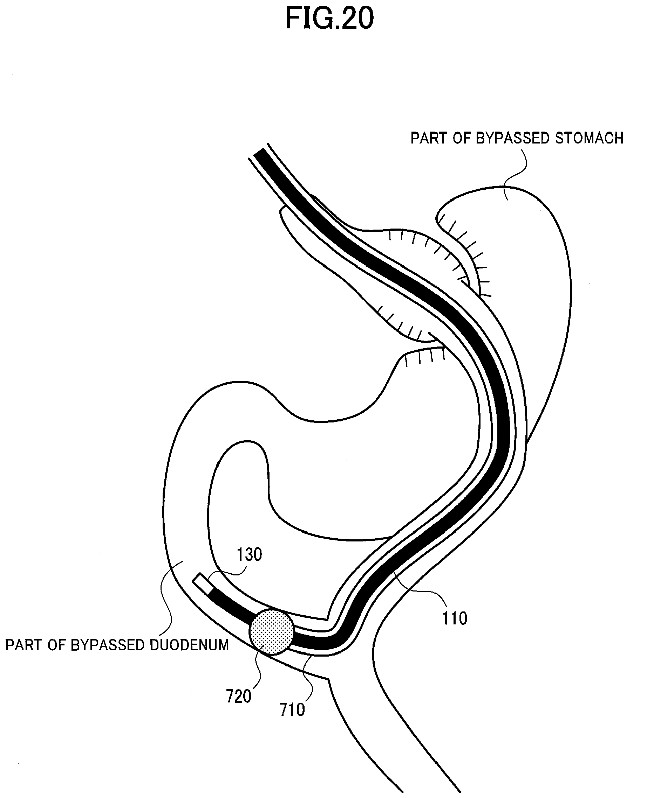

Next, a description is given for a modification using endoscopes or the like having various lengths corresponding to individual differences of patients. Since each patient has a different organ structure, it is difficult to use the same endoscope, overtube, or attachment for all patients. FIG. 20 shows an organ structure of a patient who had a gastric bypass surgery according to the Roux-en Y method, as an example of individual differences in organ structures. The Roux-en Y method is performed such that the stomach is divided into a bag connected to the esophagus and a bag connected to the duodenum, the ileum is connected to the bag connected to the esophagus, and the duodenum is connected in the middle of the ileum. The insertion distance from the mouth to the papillary portion differs between patients who had such a surgery and those who did not.

Therefore, by making the endoscope or overtube to be inserted into the body selectable for each patient by semi-reuse, single-use, or extension using attachment. Examples of reusable portions include the control device, the motor unit, the air supply/suction device, and the like. Examples of semi-reusable, single-use, or attachable portions include the endoscope, the treatment tool, the overtube, and the like. For the endoscope, the treatment tool, or the overtube, those different in length, balloon position, and the like are prepared.

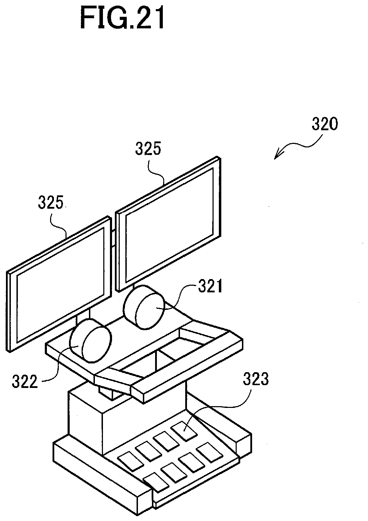

FIG. 21 shows a modification of an operation device for manually operating an electric endoscope. Although a handy operation device 300 is shown in FIG. 9, a console-type operation device 320 may be used as shown in FIG. 21. The operation device 320 includes a monitor 325 for displaying an endoscope image, an operation section 321 operated by a right hand, an operation section 322 operated by a left hand, and one or more foot switches 323. Both the operation sections 321 and 322 are capable of, for example, operation input in the vertical and horizontal directions. For example, the vertical/horizontal curving operation of the endoscope 100 is assigned to the vertical/horizontal operation of the operation section 321, the forward/backward operation and the rolling rotation operation of the endoscope 100 are assigned to the vertical/horizontal operation of the operation section 322, and the vertical operation of the raising base of the treatment tool 400 is assigned to the foot switch. The assignment of functions is not limited to this example.

As described above, the information obtainable in the ERCP procedure is only the endoscope image showing the papillary portion. Because there are individual differences in the form of the papillary portion and luminal tissue, it is difficult to specify the insertion position and the insertion direction of the cannula only based on the endoscope image. In order to more accurately assume the position of the opening and the travelling direction of the biliary duct, it is desirable to perform positioning so that the papillary portion is shown at a predetermined position in the image; however, it is difficult to adjust the position of the distal end of the endoscope because, for example, the operation performed at the base end side of the insertion section is not easily transmitted to the distal end section, or because the distal end section of the endoscope is easily displaced from the papillary portion. The U.S. Patent Application Publication No. 2017/0086929 described above discloses an example in which a robotic catheter system is applied to ERCP, but does not disclose or suggest any of the above-mentioned problems or subject matter for solving them.

Therefore, the medical system 10 of the present embodiment includes the endoscope 100 and the control device 600. In the endoscope 100, the endoscopic operation is electrically driven, thereby capturing an endoscope image. The endoscopic operation is at least one of forward and backward movement of the insertion section 110, the curving angle of the curving section 102 of the insertion section 110, and the rolling rotation of the insertion section 110. The control device 600 controls the electrically-driven endoscopic operation. After the first positioning for positioning the insertion section 110 with respect to the papillary portion of the duodenum is performed, the control device 600 controls the electrically-driven endoscopic operation based on the endoscope image, thereby performing the second positioning for positioning the distal end section 130 of the insertion section 110 with respect to the papillary portion.

According to the present embodiment, positioning of the insertion section 110 with respect to papillary portion can be done by the first positioning. Then, by performing the second positioning based on the positional relationship between the insertion section 110 thus positioned and the papillary portion, the endoscopic operation near the distal end can be more flexibly controlled by electrical driving. Further, by automatically controlling the electrical driving of the endoscopic operation or the like based on the endoscope image in the second positioning, the distal end section 130 of the insertion section 110 is automatically positioned with respect to the papillary portion. As a result, it is not necessary to perform minute position adjustment by non-electric manual operation, and it is possible to assist the cannulation procedure performed by an inexperienced operator or the like.

The endoscopic operation is described with reference to FIG. 8 in "2. Procedure Flow and Medical System According to the Present Embodiment", etc. The papillary portion of the duodenum is described with reference to FIG. 1 in "1. Explanation of ERCP", etc. The first positioning and the second positioning are described with reference to FIG. 6 in "2. Procedure Flow and Medical System According to This Embodiment", etc.

In the present embodiment, the medical system 10 may include a holding member that performs the first positioning of the insertion section 110 by holding the insertion section 110.

According to the present embodiment, since the insertion section 110 is held by the holding member in the first positioning, the electrically-driven endoscopic operation is easily transmitted from the base end side to the distal end side of the insertion section 110 in the second positioning; further, the insertion section 110 is held so that the distal end section 130 of the endoscope 100 is prevented from displacing from the papillary portion.

The holding member corresponds to the overtube 710, the balloon 720, or both described with reference to FIGS. 5, 8, 16, or 17, etc. Alternatively, the holding member may be the balloon 730 described in FIG. 18, the suction cap 740 described in FIG. 19, or the like.

In the present embodiment, the control device 600 may perform the second positioning for slightly adjusting the position of the distal end section 130 of the insertion section 110 by an endoscopic operation on a distal end side relative to the holding member holding the insertion section 110.

According to the present embodiment, in the first positioning, the position of the distal end section 130 with respect to papillary portion is roughly adjusted, and the insertion section 110 is held by the holding member. As a result, the electrically-driven endoscopic operation is accurately performed, and the distal end section 130 is prevented from displacing from the papillary portion. Therefore, in the second positioning, the position of the distal end section 130 of the endoscope 100 can be slightly adjusted with respect to the papillary portion by the endoscopic operation on a distal end side relative to the holding member.

The term "on a distal end side relative to the holding member" means a portion of the holding member closer to the distal end than a portion to be hardened, fixed, etc. to restrict the endoscopic operation. This is described above in "2. Procedure Flow and Medical System According to the Present Embodiment" with reference to FIG. 8, etc.

Further, in the present embodiment, the control device 600 may perform the second positioning for controlling the electrically-driven endoscopic operation in a manner such that the image of the papillary portion is captured at a position registered in advance on the endoscope image.

According to the present embodiment, it is possible to obtain an endoscope image in which the papillary portion is located at the position registered in advance. As a result, the papillary portion appears in a familiar position and can thus be easily compared with those in the past cases or experiences. Therefore, the operator can easily judge the position of the opening of the luminal tissue and the travelling direction of the biliary duct by viewing the endoscope image. Further, since the positioning is performed by electrical driving, it is possible to obtain an endoscope image in which the papillary portion is located at a position registered in advance without requiring the operator to perform a minute operation. In addition, the control device 600 may perform the second positioning for controlling the electrically-driven endoscopic operation in a manner such that the image of the opening of the luminal tissue is captured at a position registered in advance in the endoscope image. The operator can grasp the position of the opening of the luminal tissue by viewing the endoscope image. For example, even in a case where the opening of the luminal tissue is closed in the image of the papillary portion and therefore is difficult to be visually recognized, the operator can presume that the opening of the luminal tissue is present at a predetermined position in the endoscope image.

The position registered in advance is described above, for example, in FIG. 6 of "2. Procedure Flow and Medical System According to the Present Embodiment" or FIG. 10 of "3. Detailed Configuration Example of Medical System".

In the present embodiment, the papillary portion may include the opening of the luminal tissue. The opening of the luminal tissue may be an opening of the common duct in which the biliary duct and the pancreatic duct merge or an opening of the biliary duct, in the papillary portion of the duodenum.

In the papillary portion of the duodenum, there are various individual differences in the form of the papillary portion and the structure of the luminal tissue. According to the present embodiment, the positioning of the distal end section 130 of the endoscope 100 with respect to the papillary portion of the duodenum with various individual differences can be done by the first positioning and the second positioning. In addition, since the positioning of the papillary portion is automated, it is possible to assist the cannulation in the ERCP procedure.

The papillary portion of the duodenum, the opening of the luminal tissue, and their relationship are described, for example, in "1. Explanation of ERCP".

Further, in the present embodiment, the medical system 10 includes a treatment tool 400. The treatment tool 400 is inserted from the insertion opening 190 of the treatment tool 400 of the endoscope 100, and is raised inside the distal end section 130 to thereby protrude from the side surface of the distal end section 130. The control device 600 performs the second positioning for controlling at least one of the endoscopic operation and the raising angle of the treatment tool 400 in a manner such that the treatment tool 400 faces toward the travelling direction of the biliary duct that is presumed from the endoscope image in which the image of the papillary portion is captured.

According to the present embodiment, at least one of the endoscopic operation and the raising angle of the treatment tool 400 is automatically controlled by the second positioning so that the treatment tool 400 faces toward the travelling direction of the biliary duct. As a result, the assumption of the travelling direction of the biliary duct, which is difficult due to individual differences in the papillary portion or the like, is automated, thereby assisting the ERCP procedure performed by an inexperienced operator or the like.

The raising of the treatment tool 400 is described above in FIG. 14 of "4. Detailed Configuration Example of Each Part of Medical System", etc. The control for directing the treatment tool 400 toward the travelling direction of the biliary duct is described, for example, in FIG. 10 of "3. Detailed Configuration Example of Medical System", etc.

Further, in the present embodiment, the holding member may include a first holding member for holding the insertion section 110 with respect to the organ in which the papillary portion is present.

Specifically, the first holding member may be a member that holds the insertion section 110 with respect to the duodenum in a state in which the distal end section 130 of the endoscope 100 reaches the papillary portion of the duodenum.

Further, the first holding member may be provided closer to the base end of the insertion section 110 than the base end of the curving section 102.

Further, the first holding member may be a balloon 720 that is inflated and comes in contact with an organ, thereby holding the insertion section 110 with respect to the organ.

Since the insertion opening 190 is held in the organ by the first holding member, it becomes possible to perform the first positioning of the insertion section 110 with respect to the papillary portion in the organ, thereby flexibly controlling the endoscopic operation near the distal end by electrical driving in the subsequent second positioning.

The first holding member corresponds to the balloon 720 described above with reference to FIG. 5, 8 or 17, etc. Alternatively, the first holding member may be the balloon 730 described in FIG. 18, the suction cap 740 described in FIG. 19, or the like.

Further, in the present embodiment, the holding member may include a second holding member for holding the route of the insertion section 110 to the first holding member for holding the insertion section with respect to the organ.

The second holding member may be an overtube 710 with a variable hardness that holds the route of the insertion section 110 by being hardened.

Since the insertion opening 190 is held in the organ by the second holding member, it becomes possible to perform the first positioning of the insertion section 110 with respect to the opening of the luminal tissue in the organ, thereby flexibly controlling the endoscopic operation near the distal end section 130 by electrical driving in the subsequent second positioning.

The second holding member corresponds to the overtube 710 described with reference to FIG. 5, 8, 16, or 17, etc. The variable hardness of the overtube 710 is described above with reference to FIG. 16 in "4. Detailed Configuration Example of Each Part of Medical System", etc.

Further, in the medical system 10, the electrical driving of the curving movement of the endoscope 100 is not limited to the structure of the present embodiment. For example, it may be structured such that an attachment equipped with an electric motor is detachably attached to a curving operation knob of a non-electrically-driven endoscope. The drive control device 200 and the attachment are structured to communicate with each other, and, upon reception of a curving control signal from the drive control device 200, the attachment is driven to perform the curving. In this case, the manual control and the automatic control can be switched by attaching and detaching the attachment. It may also be arranged such that a handle capable of controlling the driving of the drive control device 200 is detachably attached to a motor unit for curving control corresponding to the drive control device 200. In this case, the manual control and the automatic control can be switched by attaching and detaching the handle.

The present embodiment may be implemented as a cannulation method as follows. More specifically, the cannulation method uses the endoscope 100. In the endoscope 100, the endoscopic operation is electrically driven, thereby capturing an endoscope image. The endoscopic operation is at least one of forward and backward movement of the insertion section 110, the curving angle of the curving section 102 of the insertion section 110, and the rolling rotation of the insertion section 110. The cannulation method includes a step of inserting the insertion section 110 of the endoscope 100 into a body. The cannulation method includes a step of performing the first positioning for positioning the insertion section 110 with respect to the papillary portion of the duodenum. The cannulation method includes, after the step of performing the first positioning, a step of performing the second positioning for positioning the distal end section 130 of the insertion section 110 with respect to the papillary portion by controlling electrically-driven endoscopic operation based on an endoscope image. The cannulation method includes, after the step of performing the second positioning, a step of performing cannulation from the papillary portion to the biliary duct.

In the present embodiment, in the step of performing the first positioning, the first positioning of the insertion section 110 may be performed by a holding member that holds the insertion section 110.

Further, in the present embodiment, in the step of performing the second positioning, the position of the distal end section 130 of the endoscope 100 may be slightly adjusted by an endoscopic operation on a distal end side relative to the holding member holding the insertion section 110.

Further, in the present embodiment, in the step of performing the second positioning, the electrically-driven endoscopic operation may be controlled so that the image of the papillary portion is captured at a position registered in advance in the endoscope image.

Further, in the present embodiment, the treatment tool 400 is inserted from the insertion opening 190 of the treatment tool 400 of the endoscope 100, and is also raised inside the distal end section 130 to thereby protrude from the side surface of the distal end section 130. In the step of performing the second positioning, it is possible to control at least one of the endoscopic operation and the raising angle of the treatment tool 400 in a manner such that the treatment tool 400 faces toward the travelling direction of the biliary duct that is presumed from the endoscope image in which the image of the papillary portion is captured.

The present embodiment may also be performed as a method of operating the medical system 10 as follows. More specifically, the method of operating the medical system 10 uses the endoscope 100. In the endoscope 100, the endoscopic operation is electrically driven, thereby capturing an endoscope image. The endoscopic operation is at least one of forward and backward movement of the insertion section 110, the curving angle of the curving section 102 of the insertion section 110, and the rolling rotation of the insertion section 110. The method of operating the medical system 10 includes a step of performing the first positioning for positioning the insertion section 110 with respect to the papillary portion of the duodenum. The method of operating the medical system 10 includes, after the step of performing the first positioning, a step of performing the second positioning for positioning the distal end section 130 of the insertion section 110 with respect to the papillary portion by controlling electrically-driven endoscopic operation based on an endoscope image. In the method of operating the medical system 10, the subject of each step is the medical system 10.

Although the embodiments to which the present disclosure is applied and the modifications thereof have been described above, the present disclosure is not limited to the embodiments and the modifications thereof, and various modifications and variations in elements may be made in implementation without departing from the spirit and scope of the present disclosure. The plurality of elements disclosed in the embodiments and the modifications described above may be combined as appropriate to form various disclosures. For example, some of all the elements described in the embodiments and the modifications may be deleted. Furthermore, elements in different embodiments and modifications may be combined as appropriate. Thus, various modifications and applications can be made without departing from the spirit and scope of the present disclosure. Any term cited with a different term having a broader meaning or the same meaning at least once in the specification and the drawings can be replaced by the different term in any place in the specification and the drawings.

## Claims

1. A medical system comprising:
an endoscope being configured to electrically drive an endoscopic operation, which is at least one of forward and backward movement of an insertion section, a curving angle of a curving section of the insertion section, and rolling rotation of the insertion section, and capture an endoscope image; and
a control device being configured to control the electrically-driven endoscopic operation,
the control device controlling the electrically-driven endoscopic operation based on the endoscope image after first positioning for positioning the insertion section with respect to a papillary portion of duodenum so as to perform second positioning for positioning a distal end section of the insertion section with respect to the papillary portion.

2. The medical system as defined in claim 1, further comprising
a holding member being configured to hold the insertion section to perform the first positioning of the insertion section.

3. The medical system as defined in claim 2, wherein
the control device performs the second positioning to slightly adjust a position of the distal end section of the insertion section by the endoscopic operation on a distal end side relative to the holding member holding the insertion section.

4. The medical system as defined in anyone of claims 1 to 3, wherein
the control device performs the second positioning to control the electrically-driven endoscopic operation so that an image of the papillary portion is captured at a position registered in advance on the endoscope image.

5. The medical system as defined in any one of claims 1 to 4, wherein
the papillary portion includes an opening of a luminal tissue, which is an opening of a common duct in which a biliary duct and a pancreatic duct merge or an opening of a biliary duct.

6. The medical system as defined in claim 5, further comprising
a treatment tool to be inserted from an insertion opening of a treatment tool of the endoscope, the treatment tool being raised inside the distal end section to protrude from a side surface of the distal end section, wherein
the control device performs the second positioning to control at least one of the endoscopic operation and a raising angle of the treatment tool so that the treatment tool faces toward a travelling direction of the biliary duct that is presumed from the endoscope image in which the image of the papillary portion is captured.

7. The medical system as defined in any one of claims 1 to 6, wherein
a/the holding member comprises a first holding member for holding the insertion section with respect to an organ in which the papillary portion is present.

8. The medical system as defined in claim 7, wherein
the first holding member is a member for holding the insertion section with respect to the duodenum in a state in which the distal end section of the endoscope reaches the papillary portion; and/or wherein
the first holding member is provided closer to a base end of the insertion section than a base end of the curving section; and/or wherein
the first holding member is a balloon that is inflated and comes in contact with the organ so as to hold the insertion section with respect to the organ.

9. The medical system as defined in claim 7, wherein
the holding member comprises a second holding member for holding a route of the insertion section to the first holding member holding the insertion section with respect to the organ.

10. The medical system as defined in claim 9, wherein
the second holding member is an overtube with a variable hardness that holds the route of the insertion section by being hardened.

11. A control method of a medical system using an endoscope that electrically drives an endoscopic operation, which is at least one of forward and backward movement of an insertion section, a curving angle of a curving section of the insertion section, and rolling rotation of the insertion section, and captures an endoscope image,
the control method of the medical system comprising,
a step of the medical system inserting the insertion section of the endoscope into a body;
a step of the medical system performing first positioning for positioning the insertion section with respect to a papillary portion of duodenum;
after the step of the medical system performing the first positioning, a step of the medical system performing second positioning for positioning a distal end section of the insertion section with respect to the papillary portion by controlling the electrically-driven endoscopic operation based on the endoscope image; and
after the step of the medical system performing the second positioning, a step of the medical system performing cannulation from the papillary portion to a biliary duct.

12. The control method of the medical system as defined in claim 11, wherein
in the step of the medical system performing the first positioning, the first positioning of the insertion section is performed by a holding member holding the insertion section.

13. The control method of the medical system as defined in claim 12, wherein
in the step of the medical system performing the second positioning, a position of the distal end section of the endoscope is slightly adjusted by the endoscopic operation on a distal end side relative to the holding member holding the insertion section.

14. The control method of the medical system as defined in anyone of claims 11 to 13, wherein
in the step of the medical system performing the second positioning, the electrically-driven endoscopic operation is controlled so that an image of the papillary portion is captured at a position registered in advance on the endoscope image.

15. The control method of the medical system as defined in anyone of claims 11 to 14, wherein
in the step of the medical system performing the second positioning, at least one of the endoscopic operation and a raising angle of the treatment tool is controlled so that a treatment tool that is inserted from an insertion opening of a treatment tool of the endoscope and is raised inside the distal end section to protrude from a side surface of the distal end section faces toward a travelling direction of a biliary duct that is presumed from the endoscope image in which an image of the papillary portion is captured.
